# EUROPEAN PATENT APPLICATION

(11) **EP 4 846 979 A1**
(43) Date of publication of application: **02.12.2026**
(21) Application number: 25745480.1
(22) Date of filing: 20.01.2025
(51) Int. Cl.: A61F 2/16

(54) **INTRAOCULAR LENS**

(30) Priority: 26.01.2024 KR 20240012739
(71) Applicant: Losec Co., Ltd., Gangwon-do 24329 (KR)
(72) Inventor: PARK, Kyoungjin, Seoul 06232 (KR)
(74) Representative: Zacco Sweden AB
(86) International application number: PCT/KR2025/001104
(87) International publication number: WO 2025/159463

(57) **Abstract**

An intraocular lens according to one aspect of the present invention comprises:
an optic part (100) and a haptic part (200),
wherein the haptic part (200) includes a connecting portion (220) connected to the optic part (100) and at least three ring-shaped loops (210) extending from the connecting portion (220).

## Description

### Technical Field

The present invention relates to an intraocular lens, and more specifically, to an accommodative intraocular lens capable of adjusting focus in response to forces transmitted from the zonules.

### Background Art

Conventional intraocular lenses have a drawback in that they do not efficiently receive forces associated with relaxation and contraction movements transmitted from the zonules within the capsular bag. To address this, the inventor of the present invention proposed an intraocular lens support structure in Korean Registered Patent No. 10-0843454. Unlike traditional capsular tension rings used to maintain the circular contour of the capsular bag during cataract surgery, the disclosed support structure resolves issues where the anterior and posterior capsules of the capsular bag adhere, causing difficulties in adjusting the lens thickness. The support structure enables efficient transmission of relaxation and contraction forces from the zonules to the intraocular lens, granting the intraocular lens functionality comparable to the natural crystalline lens, with excellent accommodative capability.

### [Prior Art Documents]

### [Patent Documents]

Prior Art Document 1: Korean Patent No. 10-0843454 "Supporter for intraocular lens"

### DISCLOSURE

### Technical Problem

As the intraocular lens support structure serves as a means to efficiently transmit the forces from the zonules to the intraocular lens, it is also necessary for the intraocular lens itself to have a structure suitable for effectively receiving these forces.

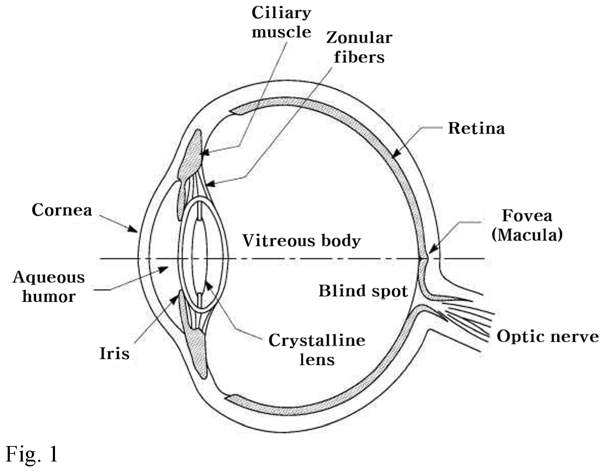

However, as illustrated in Fig. 1, the haptic part of conventional intraocular lenses typically has a simple spoke-like structure. This design makes it challenging to efficiently receive forces transmitted over the entire area where the zonules contact the capsular bag, necessitating further development in this regard.

### Technical Solution

An intraocular lens according to one aspect of the present invention comprises an optic part (100) and a haptic part (200), wherein the haptic part (200) includes a connecting portion (220) connected to the optic part (100) and at least three ring-shaped loops (210) extending from the connecting portion (220).

In this configuration, the optic part (100) includes a front surface (110a) through which light enters and a rear surface (120a) through which light exits. It is preferable for the haptic part (200) to form an angle of 2 to 10° towards the front side from a reference line dividing the front surface (110a) and the rear surface (120a).

Additionally, the front surface (110a) and the rear surface (120a) preferably have a biconvex shape.

The thickness of the haptic part is preferably 0.4 to 0.5 mm.

The area ratio of the ring-shaped loops (210) to the optic part (100) is preferably 1:2 to 1:2.5.

The total outer diameter (R) of the optic part (100) and the haptic part (200) is preferably 5 to 40% longer than the cross-sectional length of the anterior portion (410) of the capsular bag.

The intraocular lens is preferably positioned forward of the center of the capsular bag after being inserted into the capsular bag.

The haptic part (200) preferably extends forward at an angle of 2 to 10° from the reference line and then bends backward to contact the capsular bag.

The radius of curvature of the central portion of the front surface, denoted as R1, is preferably equal to or greater than the radius of curvature of the central portion of the rear surface, denoted as R2. Particularly, the R1/R2 ratio is preferably 1.1 or greater.

The length (d) of the haptic part is preferably 0.7 to 1.0 times the diameter (D) of the optic part.

An intraocular lens assembly according to another aspect of the present invention comprises:
an intraocular lens having an optic part (100), a connecting portion (220) connected to the optic part (100), and a haptic part comprising at least three ring-shaped loops (210) extending from the connecting portion (220); and
an intraocular lens support structure (300) including fixing means (330) to which the ends of the loops (210) are fixed, wherein the loops (210) contact the support structure.

The intraocular lens support structure (300) is preferably a ring-shaped structure disposed along the equatorial portion of the capsular bag. In a sectional view along the optical axis, the structure has walls with inner and outer surfaces (302, 301) that are convex toward the capsular bag. The structure includes an anterior section (310) positioned in the anterior direction along the optical axis and a posterior section (320) extending rearward from the anterior section (310) along the equatorial portion.

The optic part is preferably positioned between the anterior portion and the equatorial portion of the capsular bag.

The haptic part is preferably in close contact with the inner surface of the capsular bag, corresponding to the area where the zonules are attached.

The optic part (100) includes a front surface (110a) through which light enters and a rear surface (120a) through which light exits. The haptic part (200) preferably extends forward at an angle of 2 to 10° from the reference line dividing the front and rear surfaces and then bends backward to contact the capsular bag.

### Advantage Effects

As described above, the intraocular lens according to one aspect of the present invention includes at least three haptic parts (200) that evenly contact the inner surface (302) of the capsular bag, facilitating accurate centering of the optic part (100) without tilting when positioned within the capsular bag during surgery.

Additionally, the haptic part (200), consisting of at least three relatively large ring-shaped loops, is securely fixed in close contact with the interior of the capsular bag. During accommodation, this induces an elliptical or reverse elliptical shape change of the capsular bag, and the optic part (100) is pushed forward (towards the iris) through a highly flexible connecting portion (220), thereby achieving accommodative power comparable to that of a natural crystalline lens.

Furthermore, the small size of the optic part (100) and the thin, flexible connecting portion (220) linking the optic part (100) and the haptic part (200) allow the lens to be inserted with minimal surgical incision.

The optic part (100) is designed to move forward after being inserted into the capsular bag. Unlike conventional intraocular lenses that are typically positioned at or slightly behind the equatorial region (430) of the lens, the present invention achieves high accommodative power by maximizing the forward movement of the optic part (100) toward the anterior capsular bag in response to capsular changes.

The haptic part (200) is installed to extend forward at an angle of 2 to 10° and then bend backward to contact the capsular bag. This configuration achieves three main objectives:
1. Maximizes accommodative power by ensuring strong contact of the three haptic loops with the anterior capsular bag's internal surface, particularly at the attachment sites of the ciliary muscles.
2. Prevents tilting or decentering of the optic part (100).
3. Avoids complications such as iritis or acute glaucoma caused by excessive forward movement of the optic part toward the iris, which could obstruct the aqueous humor flow, by ensuring proper angulation.

Moreover, combining the intraocular lens with a support structure within the capsular bag of a specific structure enables higher accommodation and refractive index modulation.

### Brief Description of Drawings

Fig. 1: A schematic view of the human eye and an intraocular lens installed therein.
Fig. 2: A plan view of the intraocular lens according to one embodiment of the present invention.
Fig. 3: A cross-sectional view taken along line A-A' of Fig. 2.
Fig. 4: A cross-sectional view taken along line B-B' of Fig. 2.
Fig. 5: A schematic view showing the intraocular lens in an inserted state within the capsular bag according to one embodiment of the present invention.
Fig. 6: A plan view of the intraocular lens support structure (300) according to one embodiment of the present invention.
Fig. 7: A cross-sectional view taken along line C-C' of Fig. 6.
Fig. 8: A schematic view showing the inserted state of the intraocular lens within the capsular bag according to an embodiment of the present invention.
Fig. 9: A schematic view showing the inserted state of a reference example with the haptic part (200) oriented differently within the capsular bag.
Fig. 10: An explanatory diagram illustrating the functioning of the intraocular lens when viewing distant and near objects according to the embodiment of Fig. 8.

### Detailed Description of the embodiments

Before describing the present invention in detail, it should be understood that the terms used in this specification are intended to describe specific embodiments and are not intended to limit the scope of the invention, which is defined only by the appended claims. All technical and scientific terms used in this specification have the same meanings as commonly understood by one of ordinary skill in the art, unless otherwise defined.

Throughout the specification and claims, unless otherwise stated, the term "comprise," "comprises," or "comprising" refers to the inclusion of the stated elements, steps, or groups of elements or steps, but does not preclude the inclusion of any other elements, steps, or groups of elements or steps.

Furthermore, various embodiments of the present invention may be combined with other embodiments unless specifically stated otherwise. Particularly, any feature identified as preferable or advantageous can be combined with any other preferable or advantageous feature(s).

In the drawings, the width, length, and thickness of components may be exaggerated for convenience. Overall, the descriptions are provided from the observer's perspective. When describing one component as being "above/below" or "on/under" another component, it includes not only cases where the component is directly above or below another component but also cases where other components are interposed in between.

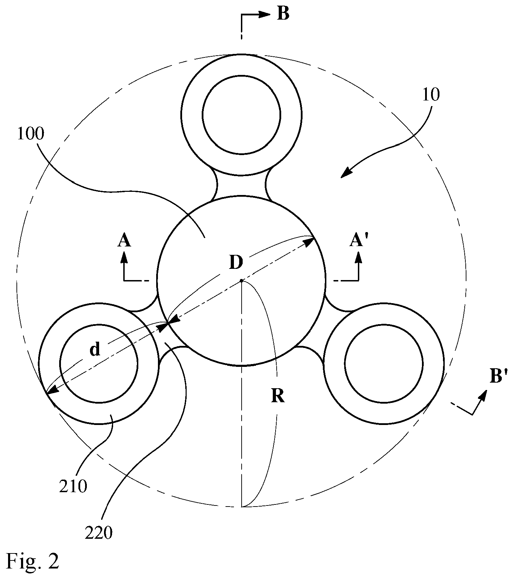

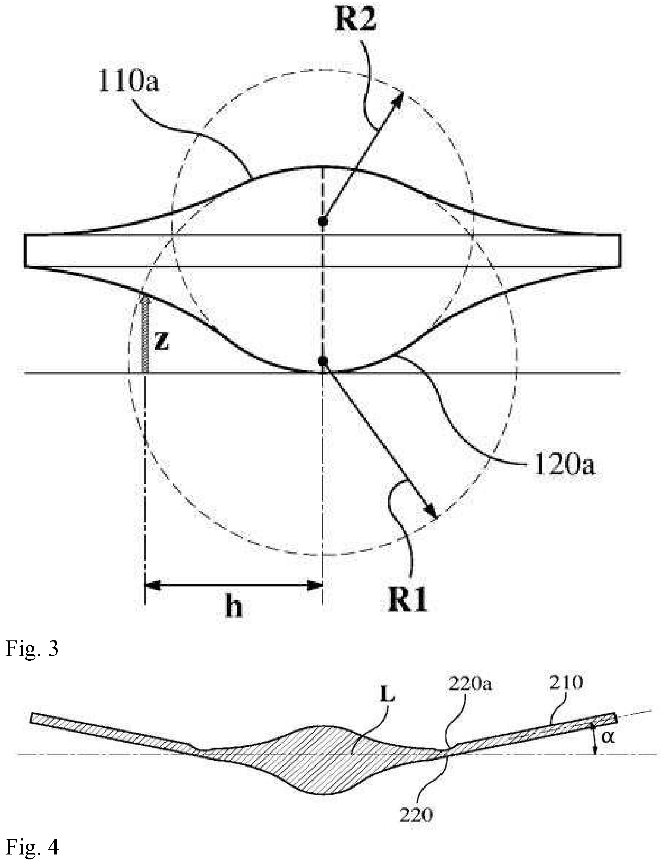

Fig. 2 is a plan view of an intraocular lens according to one embodiment of the present invention. Fig. 3 is a cross-sectional view taken along line A-A' of Fig. 2, and Fig. 4 is a cross-sectional view taken along line B-B' of Fig. 2. According to this, the intraocular lens according to one embodiment of the present invention includes an optic part (100) and a haptic part (200).

The optic part (100) forms the central portion of the intraocular lens, serving as the area through which light passes to focus, thereby performing the essential function of the intraocular lens. The optic part (100) includes a front surface (110a) where light enters and a rear surface (120a) where light exits. It is made from high-quality transparent materials to correct vision and accurately deliver images to the retina.

The optic part (100) is primarily made of acrylic, silicone, or other highly biocompatible materials, but it is not limited to these specific materials in the present invention. These materials are preferred for their transparency, high optical quality, stability within the body over long periods, and their ability to minimize allergic reactions or inflammation.

The front surface (110a) and rear surface (120a) of the optic part (100) preferably have a refractive power at the central portion that is 1.25 to 2.5 diopters (D) higher than the refractive power at the peripheral portions, resulting in a biconvex, aspheric shape with a smaller radius of curvature.

The aspheric design mimics the optical characteristics of the natural crystalline lens, providing users with a more natural visual experience. The aspheric design of the front surface (110a) enhances the refraction of light passing through the peripheral regions of the eye, while the aspheric design of the rear surface (120a) interacts with the natural fluid dynamics of the eye. This interaction helps maintain the position and stability of the artificial lens, thereby minimizing refractive errors.

It is desirable that the rear surface (120a) is configured to have an asphericity degree similar to that of the front surface (110a) (e.g., ratios such as 1.25:1.25, 1:1.25, 1.25:1, or 2.5:2.5). Mathematical equations commonly used to describe aspheric surfaces can be applied to quantify the shape of the asphericity.
(Equation 1)

Here, c represents the reciprocal of the radius of curvature at the center of the lens, k denotes the conic constant, and A1, A2, A3... are higher-order aspheric coefficients. Each aspheric coefficient indicates the degree to which the surface deviates from a spherical shape (refer to Fig. 3 for z and h). For instance, A1, as the coefficient of the r4 term, represents the extent to which the lens shape deviates from spherical near the edges, where r is large.

These coefficients, such as A1, A2, A3...... are used to describe changes in curvature from the center to the edge of the lens, thereby providing an accurate representation of the overall aspheric shape of the lens. These coefficients do not collectively describe the deviation from a sphere with a single value but serve as parameters to explain curvature differences at specific points on the lens.

For example, in the intraocular lens shown in Fig. 3, when the radius of the spherical central portion of the front surface (110a) is denoted as R1 and the radius of the spherical central portion of the rear surface (120a) is denoted as R2, R1 is equal to or greater than R2. Additionally, the curvature becomes flatter towards the edge of the optic part (100). In this case, the ratio R1/R2, expressed as R1:R2, is preferably between 1.1 and 2.

Furthermore, the intraocular lens of the present invention can be stably positioned at the center of the capsular bag (400), allowing the aspheric shape to be designed as desired to achieve the intended accommodative power. For example, the front surface (110a) and the rear surface (120a) of the optic part (100) can form aspheric surfaces that are convex at the center and become flatter toward the edges.

In conventional intraocular lenses, the diameter of the optic part (100) is generally in the range of 6 mm to 7 mm. However, in the embodiment of the present invention, the diameter of the optic part (100) is reduced to 4.3 mm to 5.5 mm, and more preferably to 4.5 mm to 4.8 mm. This reduction is intended to decrease the diameter of the optic part (100) while increasing the length of the haptic part (200). The conventional use of a 6 mm optic part (100) compensates for cases where the intraocular lens may not be precisely centered within the capsular bag (400) during surgery.

In contrast, the optic part (100) of the present invention can be reduced in size because, as described later, the haptic part (200) ensures accurate centering within the capsular bag (400). A smaller optic part (100) allows for a reduced incision size during surgery and induces more changes in the haptic part (200) during accommodation due to its relatively larger size, thereby increasing the accommodative power. Thus, the ability to reduce the size of the optic part (100) is one of the key advantages of the intraocular lens of the present invention.

The thickness of the optic part (100) is not limited but generally becomes thinner with higher refractive indices of the material, higher degrees of asphericity, and thinner base thicknesses of the optic part (100).

The haptic part (200) serves to secure the intraocular lens within the capsular bag (400), being located on the outer peripheral surface of the optic part (100) and providing stable positioning of the intraocular lens within the capsular bag (400). The haptic part (200) ensures that the lens remains correctly centered and fixed in position, even with natural movements of the eye.

The haptic part (200) is made from materials that provide both flexibility and sufficient support, such as silicone, acrylic, or polypropylene, which are stable for extended periods within the body and minimize allergic or inflammatory reactions due to their high biocompatibility.

The haptic part (200) is oriented at an angle α of approximately 2° to 10° forward relative to the reference line of the optic part (100), which divides the front surface (110a) and the rear surface (120a). This forward tilt facilitates the bending of the connecting portion (220) and enhances the contact of the loop (210) with the inner surface (302) of the anterior portion (410) of the capsular bag (400) after the intraocular lens is inserted and the optic part (100) is moved forward and fixed in position.

The haptic part (200) of the present invention includes at least three loops (210) and connecting portions (220). For example, in the case of three loops (210), each loop (210) is spaced at an angle of 120° and connected to the optic part (100) by three connecting portions (220). In the case of four loops (210), each pair of loops forms an angle of 180°, and they can be connected to the optic part (100) by four connecting portions (220) or two integrated connecting portions (220).

The loops (210) preferably have a ring shape to increase the contact area with the capsular bag (400) while remaining flexible. This design enhances force transmission by responding to the movements of the capsular bag (400). Specifically, the loops (210) deform into an elliptical shape, contacting the anterior and posterior regions of the equatorial portion (430), which improves force transmission whether used independently or in combination with the support structure (300), described later.

The diameter of the loops (210) is preferably smaller than that of the optic part (100) and is in the range of 2.8 mm to 3.2 mm. The cross-sectional shape of the ring-shaped loops is not limited and may include circular, rectangular, polygonal, or other forms. The area ratio of the loops (210) to the optic part (100) is preferably between 1:2 and 1:3, more preferably between 1:2 and 1:2.5.

The area of the optic part (100) is defined as the projected area when the optic part (100) is orthographically projected onto a plane. Similarly, the area of the loops (210) includes the internal open space and is defined by the outer shape formed by the loops (210) when orthographically projected onto a plane.

The thickness of the haptic part (200) can be the same as or similar to the attachment thickness of the connecting portions (220) on the optic part (100) and is preferably between 0.4 mm and 0.5 mm.

The connecting portions (220) are regions that connect the optic part (100) and the loops (210) and are preferably manufactured to be more flexible than the optic part (100) or the haptic part (200). For example, the connecting portions (220) can be designed with a thin thickness to allow bending. Fig. 4 illustrates a connecting portion (220) with a concave lower section and reduced thickness for enhanced flexibility.

By making the connecting portions (220) flexible, they can bend when the optic part (100) is moved forward after insertion into the capsular bag (400). This flexibility allows the loops (210) to increase their contact area with the inner surface (302) of the support structure (300), described later.

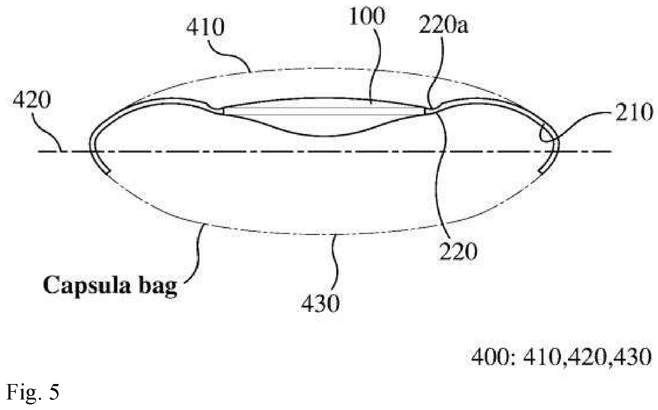

Fig. 5 illustrates a schematic view of the insertion state of an intraocular lens with four haptic parts (200) into the capsular bag (400) according to one embodiment of the present invention. For convenience, the haptic part (200) is depicted symmetrically with four loops (210); however, this should not be considered limiting.

The extension length of the intraocular lens, including the optic part (100) and haptic part (200) in a cross-sectional view, is preferably 5% to 40% longer than the extension length of the outer surface (301) of the anterior portion (410) of the capsular bag (400). This configuration allows the intraocular lens to support the capsular bag (400) from within, enabling the haptic part (200) to press against the capsular bag (400), which in turn helps maintain the tension of the capsular bag (400).

The length (d) of the haptic part (200), defined as the sum of the lengths of the connecting portion (220) and the loop (210), is preferably 0.7 to 1.0 times the diameter (D) of the optic part (100), where D/2 represents the length of the optic part (100), and ddd represents the length of the haptic part (200). It is preferable that the haptic part (200) is longer than the optic part (100). If the length falls outside this range, issues may arise, such as the optic part (100) becoming too large, increasing the required incision size during surgery, or becoming too small, failing to center properly within the capsular bag (400). This differs significantly from conventional intraocular lenses, where the diameter of the haptic part (200) is less than half the diameter of the optic part (100).

The thickness of the haptic part (200) is preferably 0.3 mm to 0.5 mm, while the concave portions of the connecting portion (220) are preferably thinner, comprising 6/10 to 9/10 of the thickness of the haptic part (200).

The materials of the optic part (100) and the haptic part (200) may be different or identical, and the materials can have varying hardness or flexibility. However, using the same material simplifies manufacturing, making it more desirable.

The intraocular lens of the present invention allows the haptic part (200) to adhere strongly and concentrically to the region of the capsular bag (400) where zonules attached to the annular ciliary muscle of the anterior portion (410) provide the strongest accommodative force.

When the natural crystalline lens accommodates for near vision, the contraction of the annular ciliary muscle transmits force through the zonules, changing the shape of the crystalline lens. To fully respond to this shape change, regardless of the internal shape of the capsular bag (400), the haptic part (200) preferably has a loop (210) design with thin edges and large openings.

The at least three loops (210) of the haptic part (200) translate the radial compressive forces of the crystalline lens into changes in the loop (210) shape. Through the connecting portion (220), acting as a lever, the loops (210) push the optic part (100) forward to provide accommodative power similar to that of the human crystalline lens, where the anterior pole protrudes during near vision accommodation.

Thus, when the intraocular lens of the present invention is inserted into the capsular bag (400), the haptic part (200) adheres first to the inner surface (302) of the anterior portion (410), corresponding to the external surface region of the lens where the zonules connected to the annular ciliary muscle are attached. Furthermore, the haptic part (200) contacts the inner surfaces (302) of the anterior portion (410), the equatorial portion (430), and the posterior portion (420) with its three or more loops (210).

It is preferable that the haptic portion (200) be entirely adhered to the inner surface (302) of the anterior portion (410), corresponding to the outer surface (301) region of the capsular bag (400) where the zonule, connected to the ciliary muscle of the anterior portion (410), attaches. Furthermore, the haptic portion (200) may partially adhere to the inner surface (302) of the posterior portion (420) through the anterior portion (410).

In conventional intraocular lenses, the optic part (100) is fixed at or slightly behind the equatorial portion (430), and the haptic part (200) is rod-shaped, piercing the equatorial portion (430). In contrast, the optic part (100) of the present invention is positioned anteriorly in the capsular bag (400), allowing the haptic part (200) to strongly adhere to the area where the zonules connected to the annular ciliary muscle provide the greatest accommodative force.

The intraocular lens of the present invention may optionally be used with an intraocular lens support structure (300) to enhance sensitivity to forces transmitted through the capsular bag (400). However, the intraocular lens can also be used independently without the support structure (300).

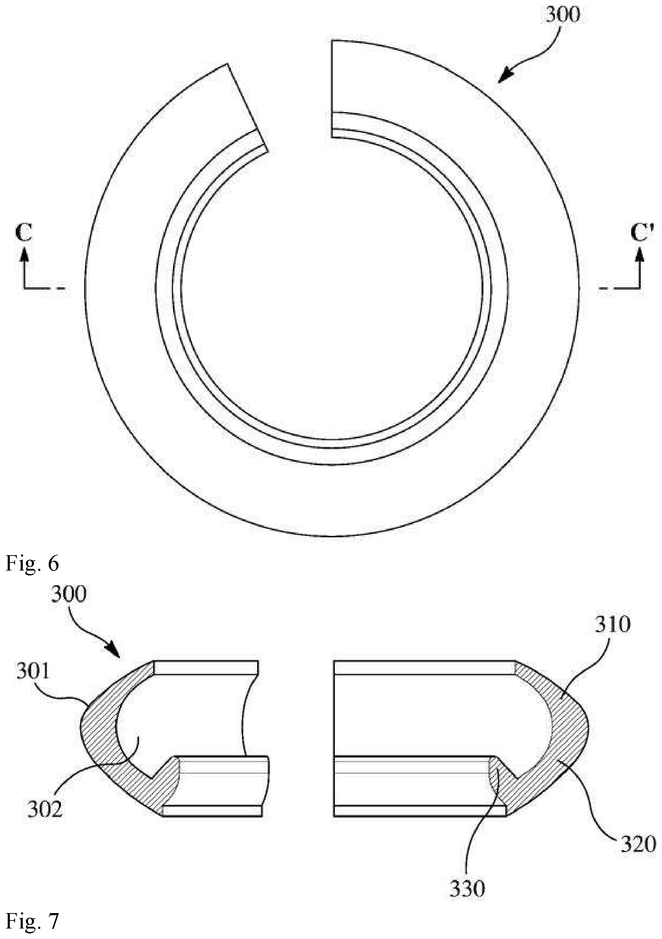

Fig. 6 illustrates a plan view of the intraocular lens support structure (300) according to one embodiment of the present invention, and Fig. 7 provides a cross-sectional view taken along line C-C' of Fig. 6.

In the present invention, the connection support structure (300) maintains the shape of the capsular bag (400) while preventing the adhesion of the anterior and posterior capsules of the capsular bag (400). Simultaneously, it efficiently transmits the relaxation and contraction forces of the zonules to the intraocular lens, enabling the intraocular lens to provide vision with depth perception, akin to that of a natural crystalline lens.

The connection support structure (300) also includes a fixing means (330) for the haptic part (200), ensuring accurate central positioning of the intraocular lens within the capsular bag (400). Additionally, the fixing means (330) facilitates forward movement of the intraocular lens in response to forces transmitted from the zonules, thereby enhancing vision clarity when viewing distant objects.

The connection support structure (300) is inserted into the interior space of the capsular bag (400) during cataract surgery before the intraocular lens. The outer surface (301) of the connection support structure (300) contacts and is secured to the equatorial portion (430) of the capsular bag (400). Once the intraocular lens is inserted, the haptic part (200) of the intraocular lens rests on the inner surface (302) of the connection support structure (300).

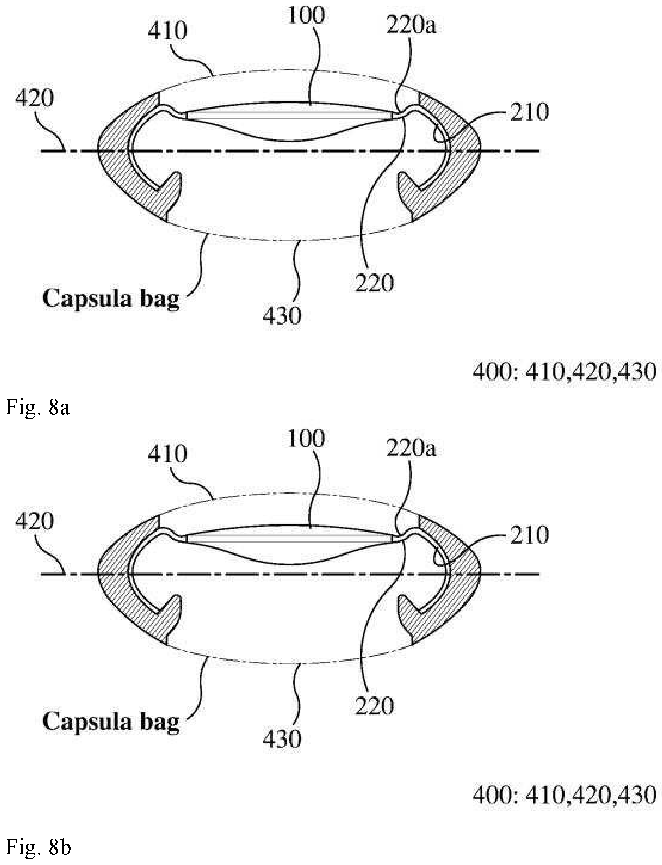

The connection support structure (300) is an open or closed ring-shaped structure. As shown in Fig. 8, in a cross-sectional view along a virtual plane in the optical axis direction (Y-direction) of the lens, both the inner surface (302) and outer surface (301) have convex walls. The walls include, in cross-sectional view, an anterior section (310), a posterior section (320), and a fixing means (330), with the equator of the support structure serving as the boundary.

The anterior section (310) is the region located anterior to the equatorial line, which connects the apexes of the convex wall of the connection support structure (300). The posterior section (320) extends from the anterior section (310) and is located posterior to the equatorial line. The fixing means (330) is provided in a specific region of the wall and serves to secure the ends of the haptic part (200) of the intraocular lens.

A preferred example of the fixing means (330) is a bent section. The bent section extends inward from the posterior section (320). The ends of the haptic part (200) of the intraocular lens may rest at the point where the inner surface (302) of the posterior section (320) meets the inner surface (302) of the bent section or may rest directly on the inner surface (302) of the bent section.

The meeting point of the inner surface (302) of the posterior section (320) and the inner surface (302) of the bent section forms an area where the end of the haptic part (200) can rest securely. Even if the haptic part (200) is longer, its end can extend beyond this point and rest directly on the inner surface (302) of the bent section.

At the point where the inner surface (302) of the posterior section (320) meets the inner surface (302) of the bent section, the inner surface (302) of the posterior section (320) is curved, while the inner surface (302) of the bent section is planar. In this case, the angle formed between the tangent of the inner surface (302) of the posterior section (320) and the planar inner surface (302) of the bent section, as observed in cross-section, is preferably between 45° and 110°, and more preferably between 60° and 100°.

When the inner surface (302) of the posterior section (320) and the inner surface (302) of the bent section are both curved near their meeting point, the angle formed between the tangent of the inner surface (302) of the posterior section (320) and the tangent of the inner surface (302) of the bent section, as observed in cross-section, is preferably between 45° and 110°, and more preferably between 60° and 90°.

If the angle exceeds these ranges, the haptic leg (200) of the intraocular lens may fail to rest securely at the meeting point of the inner surface (302) of the posterior section (320) and the inner surface (302) of the bent section. Conversely, if the angle is below these ranges, the haptic leg (200) may extend past the meeting point and fail to rest on the inner surface (302) of the bent section, particularly if the haptic leg (200) is long.

Another example of the fixing means (330) is a protrusion provided on the inner surface (302) of the wall of the connection support structure (300). Unlike the bent section, the protrusion extends directly from the inner surface of the posterior section (320) along the ring shape. However, like the bent section, it functions to secure the haptic leg (200) of the intraocular lens.

Yet another example of the fixing means (330) involves forming an additional seating groove in the bent section. The seating groove provides enhanced stability for the end of the haptic leg (200).

The connection support structure (300) preferably includes an open section to facilitate insertion during surgery. The open section is preferably angled between 1° and 30° at the center. If the open angle is less than 1°, insertion through a small surgical incision becomes difficult. If the open angle exceeds 30°, the connection support structure (300) may lose its ability to effectively support the intraocular lens.

When the connection support structure (300) is used, its overall diameter can be manufactured in the range of 9.0 mm to 12 mm, depending on the size of the eye.

The inner surface (302) is a smooth surface that allows the haptic part (200) of the intraocular lens to glide easily, ensuring proper alignment. The bent section of the fixing means is designed to securely hold the end of the haptic part (200) in place, preventing detachment and providing stable fixation.

The outer surface (301) is the surface that contacts the inner surface (302) of the capsular bag (400). It preferably contacts the capsular bag (400) at at least two points. To effectively transmit forces applied to the capsular bag (400), it is desirable for at least 1/2 to the entirety of the cross-sectional length of the outer surface (301) to be in contact with the capsular bag (400). Additionally, at least half of the contact area should include the region near the equator where the zonules are attached. This configuration ensures efficient transmission of forces from the zonules.

Fig. 8 illustrates a schematic view of the intraocular lens inserted within the capsular bag (400) according to an embodiment of the present invention.

In this embodiment, when the intraocular lens is inserted into the capsular bag (400), the optic part (100), being small, and the loops (210), being highly flexible, allow insertion through a small incision. Once inserted, the intraocular lens is evenly spread within the capsular bag (400) due to the haptic part (200) having a thickness similar to that of the optic part (100). This ensures that the optic part (100) is positioned at the center, and the ends of the haptic part (200) slightly bend and partially contact the inner wall (302) of the capsular bag (400).

Subsequently, when the optic part (100) is moved forward, the flexibility of the connecting portion (220) causes it to angle forward by approximately 2° to 10° and then bend backward. This means the haptic part (200) initially bends forward and then bends backward, making significant contact with the anterior inner wall (302) of the capsular bag (400).

As a result, the forces transmitted to the capsular bag (400) can be effectively received, and the optic part (100) is stably positioned at the anterior surface (110a). This allows the intraocular lens to better mimic the natural crystalline lens within the eye's optical system, providing a natural outcome in terms of light refraction and focal precision.

The forward angulation of approximately 2° to 10° of the connecting portion (220) provides several additional benefits:
1. The three rings of the haptic part (200) make strong contact with the anterior inner wall (302) of the capsular bag (400), particularly in the region where the zonules attached to the annular ciliary muscle of the anterior portion (410) are located. This enhances accommodative power, which is greater in the anterior inner wall (302) compared to the posterior inner wall.
2. The forward angulation helps prevent tilting or decentering of the optic part (100) of the intraocular lens, ensuring stable alignment within the capsular bag (400).
3. In cases where there is no angulation or the angulation is directed posteriorly, the anterior pole of the optic part (100) of the intraocular lens is generally flatter than that of a natural crystalline lens. Excessive accommodation could cause the anterior pole of the optic part (100) to move excessively towards the anterior portion (410) (near the iris), leading to contact with the iris and potentially causing iritis. In more severe cases, such contact may obstruct the flow of aqueous humor, resulting in acute angle-closure glaucoma. The forward angulation effectively eliminates such risks.

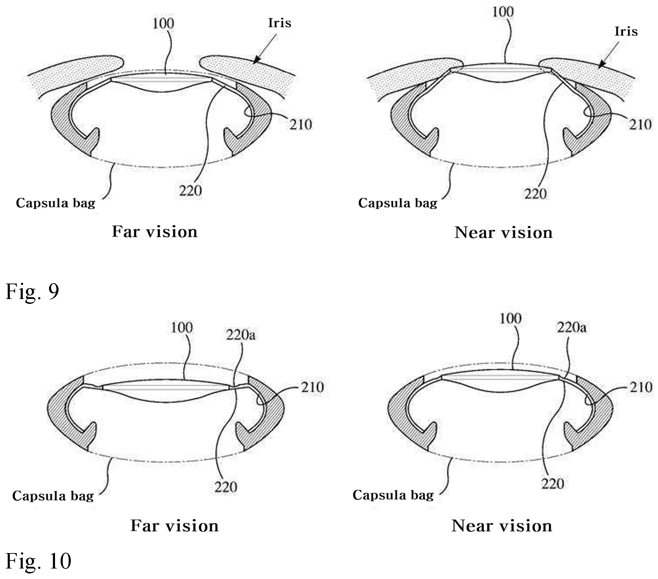

Fig. 9 illustrates the insertion state of a reference intraocular lens within a capsular bag (400) when used with a connection support structure (300). While the reference intraocular lens shares the same material and shape as the intraocular lens of the present invention, the direction of the bending of the haptic part (200) is not angled forward by 2° to 10°. Instead, it lacks angulation or exhibits posterior angulation.

In such cases, the centering of the optic part (100) is weakened. Furthermore, during near or far vision accommodation, there is an increased risk of developing iritis or acute angle-closure glaucoma.

Fig. 10 illustrates the operation of the intraocular lens when the haptic part (200) of the lens is seated on the connection support structure (300) and used for near and far vision. When the connection support structure (300) is used, its maximum thickness falls within the range of 0.35≤x≤1. Due to the limited internal space, the legs of the haptic part (200) cannot fully extend but instead bend until they reach the bent sections of the connection support structure (300).

As shown in Fig. 10, the legs of the haptic part (200) extend along the inner surface (302) of the connection support structure (300) and are ultimately seated at the bent sections. This configuration allows the haptic part (200) to effectively receive forces transmitted through the connection support structure (300), ensuring stability. Additionally, the ends of the haptic part (200) are secured at the bent sections, facilitating improved centering of the intraocular lens.

When the connection support structure (300) with the fixing means (330) is used, the distance from the center of the optic part (100) to the end of the haptic part (200), denoted as D/2+d, is preferably longer than the peripheral distance from the apex of the anterior portion (410) of the capsular bag (400) to the fixing means (330). More specifically, this distance is preferably 105% to 120% of the distance to the fixing means (330). This ensures that the ends of the three rings of the haptic part (200) adequately contact the fixing means (330), facilitating efficient force transmission and centering within the capsular bag (400).

By designing the total length from the center of the optic part (100) to the ends of the rings to exceed the extension length from the apex of the anterior portion (410) of the capsular bag (400) to the bent end of the inner wall of the connection support structure (300), the optic part (100) is positioned anterior to the equatorial line.

Positioning the optic part (100) anterior to the equatorial line allows the forces transmitted from the capsular bag (400) to the connection support structure (300) to induce deformation of the haptic part (200) when viewing near objects. This forward deformation causes the optic part (100) to move anteriorly, thereby enhancing accommodative power and maximizing corrective effects.

In contrast, conventional intraocular lenses (IOLs) are positioned at or posterior to the equatorial portion (430). This results in the intraocular lens being fixed in a slightly posteriorly convex configuration. When forces are transmitted to the intraocular lens in this configuration, it tends to move slightly posteriorly, reducing accommodative power. However, in the present embodiment, the forward movement of the optic part (100) enables significantly higher accommodative power.

When viewing far distance, the optic part (100) moves slightly posteriorly, reversing the mechanism observed during anterior movement.

The features, structures, and effects described in each of the above embodiments may be combined or modified by those skilled in the art to create additional embodiments. Therefore, such combinations and modifications should be considered within the scope of the present invention.

### Industrial Applicability

The present invention can be utilized in intraocular lenses for vision correction.

### [Description of Reference Numerals]

- **100**: Optic part
- **110a**: Front surface
- **120a**: Rear surface
- **200**: Haptic part
- **210**: Loop
- **220**: Connecting portion
- **300**: Connection support structure
- **301**: Outer surface
- **302**: Inner surface
- **310**: Anterior section
- **320**: Posterior section
- **330**: Fixing means
- **400**: Capsular bag
- **410**: Anterior portion
- **420**: Posterior portion
- **430**: Equatorial portion

## Claims

1. An intraocular lens comprising:
an optic part (100); and
a haptic part (200),
wherein the haptic part (200) comprises a connecting portion (220) connected to the optic part (100) and at least three ring-shaped loops (210) extending from the connecting portion (220).

2. The intraocular lens according to claim 1, wherein the optic part (100) includes a front surface (110a) through which light enters and a rear surface (120a) through which light exits, and the haptic part (200) forms an angle of 2 to 10° towards the front side from a reference line dividing the front surface (110a) and the rear surface (120a).

3. The intraocular lens according to claim 2, wherein both the rear surface (120a) and the front surface (110a) have an aspheric shape with a central refractive power 1.25 to 2.5 diopters higher than the peripheral refractive power.

4. The intraocular lens according to claim 3, wherein both the rear surface (120a) and the front surface (110a) have a biconvex shape.

5. The intraocular lens according to claim 1, wherein the thickness of the haptic part is 0.4 to 0.5 mm.

6. The intraocular lens according to claim 1, wherein the area ratio of the ring-shaped loops (210) to the optic part (100) is 1:2 to 1:2.5.

7. The intraocular lens according to claim 1, wherein the total outer diameter (R) of the optic part (100) and the haptic part (200) is 5 to 40% longer than the cross-sectional length of the anterior portion (410) of a capsular bag (400).

8. The intraocular lens according to claim 1, wherein the length (d) of the haptic part is 0.7 to 1.0 times the diameter (D) of the optic part.

9. The intraocular lens according to claim 1, wherein the intraocular lens, after being inserted into a capsular bag (400), is positioned forward of the center of the capsular bag (400).

10. The intraocular lens according to claim 2, wherein the haptic part (200), after being inserted into a capsular bag (400), extends forward at an angle of 2 to 10° relative to the reference line, and then bends backward to contact the capsular bag (400).

11. The intraocular lens according to claim 2, wherein the radius of curvature of the central portion of the front surface (110a), denoted as R1, is equal to or greater than the radius of curvature of the central portion of the rear surface (120a), denoted as R2.

12. The intraocular lens according to claim 11, wherein the front surface (110a) and the rear surface (120a) of the optic part form aspheric shapes that are convex at the center and flatten towards the edges.

13. The intraocular lens according to claim 11, wherein the R1/R2 ratio is between 1.1 and 2.

14. The intraocular lens according to claim 8, wherein the length (d) of the haptic part is greater than half the diameter (D/2) of the optic part.

15. An intraocular lens assembly for insertion into a capsular bag comprising anterior, equatorial, and posterior portions, the assembly comprising:
an intraocular lens having an optic part (100), a connecting portion (220) connected to the optic part (100), and at least three ring-shaped loops (210) extending from the connecting portion (220) as a haptic part; and
an intraocular lens support structure (300) including fixing means (330) to which the ends of the loops (210) are fixed, wherein the loops (210) contact the support structure.

16. The intraocular lens assembly according to claim 15, wherein the intraocular lens support structure (300) is a ring-shaped structure disposed along the equatorial portion of the capsular bag (400), and in a sectional view along the optical axis, the inner surface (302) and outer surface (301) of the structure are convex toward the capsular bag (400), the structure including an anterior section (310) positioned in the anterior direction along the optical axis and a posterior section (320) extending rearward from the anterior section (310) and positioned in the posterior direction along the equatorial portion.

17. The intraocular lens assembly according to claim 15, wherein the optic part is positioned between the anterior portion and the equatorial portion of the capsular bag (400).

18. The intraocular lens assembly according to claim 16, wherein the haptic part (200) is primarily in contact with the inner surface of the anterior portion (410) of the capsular bag (400), corresponding to the area where the zonules are connected.

19. The intraocular lens assembly according to claim 18, wherein the optic part (100) includes a front surface (110a) through which light enters and a rear surface (120a) through which light exits, and the haptic part (200) extends forward at an angle of 2 to 10° from a reference line dividing the front surface (110a) and the rear surface (120a), and then bends backward to contact the capsular bag (400).

20. The intraocular lens assembly according to claim 19, wherein the sum of half the diameter of the optic part (D/2) and the length (d) of the haptic part from the optic center to the tip is longer than the outer circumference of the capsular bag (400) from the apex of the anterior portion to the fixing means.
